# EUROPEAN PATENT APPLICATION

(11) **EP 3 441 713 A1**
(43) Date of publication of application: **13.02.2019**
(21) Application number: 17779161.3
(22) Date of filing: 05.04.2017
(51) Int. Cl.: G01B 11/00, C12M 1/34, G02B 7/28, G02B 21/00, G01N 1/28

(54) **BOTTOM SURFACE POSITION DETECTION DEVICE, IMAGE ACQUISITION APPARATUS, BOTTOM SURFACE POSITION DETECTION METHOD, AND IMAGE ACQUISITION METHOD**

(30) Priority: 08.04.2016 JP 2016077786
(71) Applicant: SCREEN Holdings Co., Ltd., Kyoto-shi, Kyoto 602-8585 (JP)
(72) Inventor: SHIBATA Fumiharu, Kyoto-shi Kyoto 602-8585 (JP); IKEZAWA Ryosuke, Kyoto-shi Kyoto 602-8585 (JP); KOKUBO Masahiko, Kyoto-shi Kyoto 602-8585 (JP)
(74) Representative: Goddar, Heinz J.
(86) International application number: PCT/JP2017/014184
(87) International publication number: WO 2017/175783

(57) **Abstract**

This image acquisition apparatus (2) includes a bottom surface position detection apparatus (1), and acquires an image of biological cells disposed in wells of a vessel. The bottom surface position detection apparatus (1) includes a holder (10), a light irradiator (20), a detector (30), and a controller (70). The holder (10) horizontally holds a vessel (9). The light irradiator (20) irradiates a bottom portion of the vessel (9) with a light beam. The detector (30) detects a first reflected light beam reflected from a first bottom surface that is a bottom surface of the vessel (9) and a second reflected light beam reflected from a second bottom surface that is a bottom surface of the wells. The controller (71) calculates the position of the second bottom surface, based on a first peak position of the first reflected light beam and a second peak position of the second reflected light beam both detected by the detector. Thus, the bottom surface position of the wells in the vessel is detected even if the vessel has the bottom portion uneven in thickness or has a curved bottom surface.

## Description

### Technical Field

The present invention relates to a bottom surface position detection apparatus for detecting a bottom surface position of a light-permeable vessel, an image acquisition apparatus including the bottom surface position detection apparatus, a bottom surface position detection method for detecting a bottom surface position of a light-permeable vessel, and an image acquisition method including the bottom surface position detection method.

### Background Art

For acquisition of images of biological cells cultivated in a culture vessel by means of an image acquisition apparatus, it is necessary to find a focal position suitable for observation of the cells. A technique for focusing an objective lens on such specimens is disclosed in Patent Literature 1, for example. An autofocus apparatus disclosed in Patent Literature 1 includes a detection means, a focal plane movement means, and a control means. The detection means has a light source and a detector. The detector detects light emitted from the light source and then reflected back from a surface and an interface of a transparent plate member through the objective lens. The focal plane movement means moves a focal plane of the objective lens and the specimens relative to each other in an optical axis direction. The control means controls the focal plane movement means, based on an output from the detector.

### Citation List

### Patent Literature

Patent Literature 1: Japanese Patent No. 4642709

### Summary of Invention

### Technical Problem

A well plate which is a plastic molded article having an array of depressions or wells is often used as the culture vessel for the cells. Such a well plate has a bottom portion uneven in thickness or has a curved bottom surface in some cases due to plastic molding problems. For this reason, an attempt to use an in-focus position obtained in one well to observe the cells in other wells gives rise to a problem such that images with blurred focus result. Thus, there has been a need for a technique for detecting an in-focus position for each of the wells in consideration of the uneven thickness of the bottom portion of the well plate and the curvature of the bottom surface thereof. To this end, it is necessary to detect the position of the bottom surface (an upper surface of a bottom plate portion) of each of the wells.

In view of the foregoing, it is an object of the present invention to provide a technique capable of detecting a bottom surface position of wells of a vessel even if the vessel has a bottom portion uneven in thickness or has a curved bottom surface.

### Solution to Problem

To solve the aforementioned problem, a first aspect of the present invention is intended for a bottom surface position detection apparatus for detecting a bottom surface position of a light-permeable vessel. The bottom surface position detection apparatus comprises: a holder for horizontally holding the vessel; a light irradiator for irradiating a bottom portion of the vessel with a light beam; a detector for detecting the light beam emitted from the light irradiator and then reflected from the vessel; and a controller, the vessel having at least one vertically depressed well, the detector detecting a first reflected light beam reflected from a first bottom surface that is a bottom surface of the vessel and a second reflected light beam reflected from a second bottom surface that is a bottom surface of the well, the controller calculating the position of the second bottom surface, based on a first peak position of the first reflected light beam and a second peak position of the second reflected light beam both detected by the detector.

A second aspect of the present invention is intended for the bottom surface position detection apparatus of the first aspect, wherein the light irradiator directs the light beam obliquely upwardly toward the bottom portion of the vessel.

A third aspect of the present invention is intended for an image acquisition apparatus for acquiring an image of biological cells disposed in the well of the vessel. The image acquisition apparatus comprises: a bottom surface position detection apparatus as recited in Claim 1 or 2; an imaging part for taking an image of the cells in the well; an image processing part for processing the image taken by the imaging part; a vertical movement mechanism for moving the holder in a vertical direction; an autofocus mechanism for adjusting a focal position of the imaging part with respect to the cells; and a horizontal movement mechanism for moving the imaging part, the light irradiator, and the detector in a horizontal direction relative to the vessel, the controller controlling the vertical movement mechanism while referring to a detection result of the detector, and controlling the autofocus mechanism after the focal position of the imaging part is adjusted to the second bottom surface.

A fourth aspect of the present invention is intended for the image acquisition apparatus of the third aspect, wherein the controller stores the position of the second bottom surface at a certain position as an autofocus reference position to control the autofocus mechanism, based on the autofocus reference position and the second peak position detected from the detector, after controlling the horizontal movement mechanism.

A fifth aspect of the present invention is intended for the image acquisition apparatus of the third or fourth aspect, wherein the controller stores a difference between the first peak position and the second peak position as an alternative correction value to control the autofocus mechanism, based on the first peak position detected from the detector and the alternative correction value, after controlling the horizontal movement mechanism.

A sixth aspect of the present invention is intended for a bottom surface position detection method for detecting a bottom surface position of a light-permeable vessel including at least one vertically depressed well. The bottom surface position detection method comprises the steps of: a) horizontally holding the vessel; b) irradiating a bottom portion of the vessel with a light beam from a light irradiator; c) detecting the light beam impinging upon the bottom portion of the vessel and then reflected from the vessel by means of a detector; and d) calculating the bottom surface position of the vessel, the step c) detecting a first reflected light beam reflected from a first bottom surface that is a bottom surface of the vessel and a second reflected light beam reflected from a second bottom surface that is a bottom surface of the well, the step d) calculating the position of the second bottom surface, based on a first peak position of the first reflected light beam and a second peak position of the second reflected light beam both detected by the step c).

A seventh aspect of the present invention is intended for the bottom surface position detection method of the sixth aspect, wherein the step c) directs the light beam obliquely upwardly toward the bottom portion of the vessel.

An eighth aspect of the present invention is intended for an image acquisition method including a bottom surface position detection method as recited in Claim 6 or 7 and for acquiring an image of biological cells disposed in the well of the vessel. The image acquisition method comprises the steps of: e) taking an image of the cells in the well by means of an imaging part; and f) processing the image taken by the imaging part, the step d) moving the vessel in a vertical direction while referring to a detection result in the step c), and adjusting a focal position of the imaging part with respect to the cells by means of an autofocus mechanism after the focal position of the imaging part is adjusted to the second bottom surface.

A ninth aspect of the present invention is intended for the image acquisition method of the eighth aspect, wherein the step d) stores the position of the second bottom surface at a certain position as an autofocus reference position to control the autofocus mechanism, based on the autofocus reference position and the second peak position detected from the detector, after controlling the horizontal movement mechanism.

A tenth aspect of the present invention is intended for the image acquisition method of the eighth or ninth aspect, wherein the step d) includes the step of storing a difference between the first peak position and the second peak position as an alternative correction value to control the autofocus mechanism, based on the first peak position detected in the step c) and the alternative correction value.

### Advantageous Effects of Invention

According to the first to tenth aspects of the present invention, the bottom surface position of the well is detected even if the vessel has the bottom portion uneven in thickness or has a curved bottom surface.

In particular, according to the second and seventh aspects, the difference between the first peak position and the second peak position is greater. Thus, the second bottom surface position is calculated more precisely.

In particular, according to the third and eighth aspects, the imaging part is focused on the cells disposed in the well even if the vessel has the bottom portion uneven in thickness or has a curved bottom surface. This achieves the acquisition of a sharp image of the cells in the well.

In particular, according to the fourth and ninth aspects of the present invention, the autofocus mechanism is controlled by using the position of the second bottom surface at a certain position as the autofocus reference position. This allows the imaging part to be focused easily on the cells in other positions.

In particular, according to the fifth and tenth aspects of the present invention, the autofocus process is performed even if the second reflected light beam is not obtained.

### Brief Description of Drawings

Fig. 1 is a perspective view of a well plate.
Fig. 2 is a partial sectional view of the well plate.
Fig. 3 is a schematic diagram of a bottom surface position detection apparatus.
Fig. 4 is a graph showing a distribution of the amount of reflected light entering a CMOS sensor.
Fig. 5 is a diagram conceptually showing a configuration of an image acquisition apparatus.
Fig. 6 is a view showing the well plate and its vicinities in the image acquisition apparatus.
Fig. 7 is a view showing a course of a scanning process by means of the image acquisition apparatus.
Fig. 8 is a flow diagram showing a procedure for an image acquisition process.
Fig. 9 is a flow diagram showing a procedure for an AF reference position setting process.
Fig. 10 is a graph showing a distribution of the amount of reflected light entering the CMOS sensor.
Fig. 11 is a graph showing a distribution of the amount of reflected light entering the CMOS sensor.
Fig. 12 is a graph showing a distribution of the amount of reflected light entering the CMOS sensor.
Fig. 13 is a graph showing a distribution of the amount of reflected light entering the CMOS sensor.
Fig. 14 is a flow diagram showing a procedure for the scanning process.
Fig. 15 is a flow diagram showing details on an autofocus process.
Fig. 16 is a graph showing a distribution of the amount of reflected light entering the CMOS sensor according to a modification.
Fig. 17 is a graph showing a distribution of the amount of reflected light entering the CMOS sensor according to the modification.

### Description of Embodiments

An embodiment according to the present invention will now be described with reference to the drawings.

### <1. Well Plate>

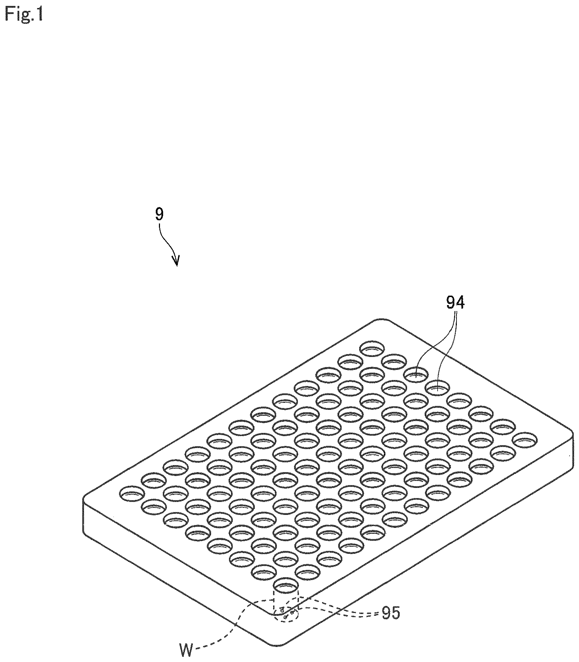

Fig. 1 is a perspective view of a well plate 9 to be set in a bottom surface position detection apparatus 1 according to the present embodiment. As shown in Fig. 1, the well plate 9 is a generally plate-shaped specimen container. An example of the material of the well plate 9 used herein includes a transparent plastic which allows light to pass therethrough. The well plate 9 has a plurality of depressions or wells W depressed downwardly from an upper surface thereof.

As shown in Fig. 1, the wells W are arranged regularly in a horizontal direction. Biological cells 95 to be observed are held with a culture solution 94 in each of the wells W. The culture solutions 94 in the respective wells W may be culture solutions of the same type or different culture solutions to which compounds with different concentrations and different compositions are added. The number of wells W included in the well plate 9 may be different from that in the example of Fig. 1. The shape of the wells W may be circular as seen in top plan view as shown in Fig. 1 or other shapes such as a rectangular shape.

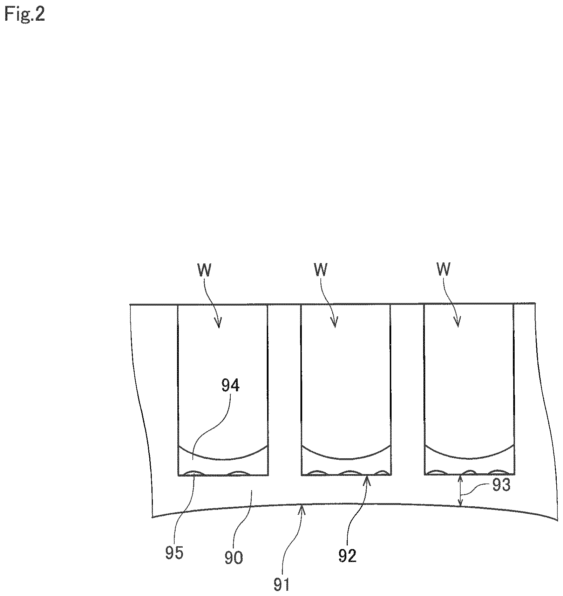

Fig. 2 is a partial sectional view of the well plate 9. As shown in Fig. 2, the well plate 9 includes a bottom portion having a first bottom surface 91 that is a bottom surface of the well plate 9 and a second bottom surface 92 that is a bottom surface of the wells W. The first bottom surface 91 is a lower surface of a bottom plate portion 90 of the well plate 9. The second bottom surface 92 is an upper surface of the bottom plate portion 90 of the well plate 9. Thus, a vertical width between the first bottom surface 91 and the second bottom surface 92 is a thickness 93 of the bottom plate portion 90. As shown in Fig. 2, the first bottom surface 91 of the well plate 9 is slightly curved due to plastic molding problems. Also, the thickness 93 of the bottom plate portion 90 of the wells W is uneven. The unevenness of the thickness 93 is in the range of tens of micrometers to a hundred and tens of micrometers.

### <2. Configuration of Bottom Surface Position Acquisition Apparatus>

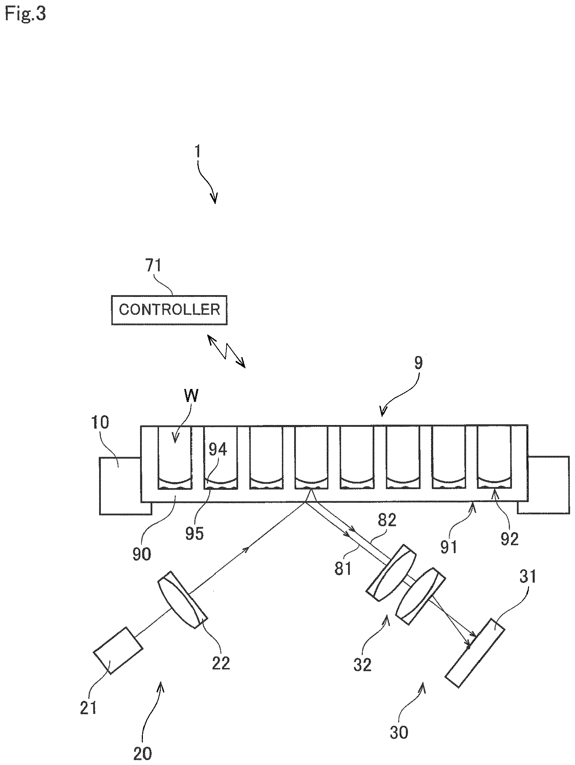

Fig. 3 is a schematic diagram of the bottom surface position detection apparatus 1 according to the present embodiment. This bottom surface position detection apparatus 1 is an apparatus for detecting the bottom surface position of the well plate 9. As shown in Fig. 3, the bottom surface position detection apparatus 1 includes a holder 10, a light irradiator 20, a detector 30, and a controller 71.

The holder 10 is a table for holding the well plate 9 thereon. The well plate 9 in a horizontal attitude with the first bottom surface 91 downside is set on the holder 10. The holder 10 holds a peripheral portion of the well plate 9. Thus, the bottom portion of the well plate 9 is not covered with the holder 10 but is exposed.

The light irradiator 20 is a mechanism for directing a light beam toward the bottom portion of the well plate 9. The light irradiator 20 according to the present embodiment includes a laser diode 21 and a lens 22. As shown in Fig. 3, the light irradiator 20 according to the present embodiment directs a laser light beam obliquely upwardly from below the well plate 9 toward the bottom portion of the well plate 9. As indicated by an arrow in Fig. 1, the laser light beam emitted from the laser diode 21 is collected by the lens 22, and travels toward the first bottom surface 91. The laser light beam that reaches the first bottom surface 91 is divided into a first reflected light beam 81 reflected from the first bottom surface 91 and a second reflected light beam 82 refracted and travels from the first bottom surface 91 into the bottom plate portion 90 and then reflected from the second bottom surface 92.

The detector 30 is a mechanism for detecting the laser light beam emitted from the light irradiator 20 and then reflected from the bottom portion of the well plate 9. The detector 30 according to the present embodiment includes a CMOS sensor 31 and a pair of lenses 32. The first reflected light beam 81 reflected from the first bottom surface 91 and the second reflected light beam 82 reflected from the second bottom surface 92 are collected by the pair of lenses 32 and then enter the CMOS sensor 31.

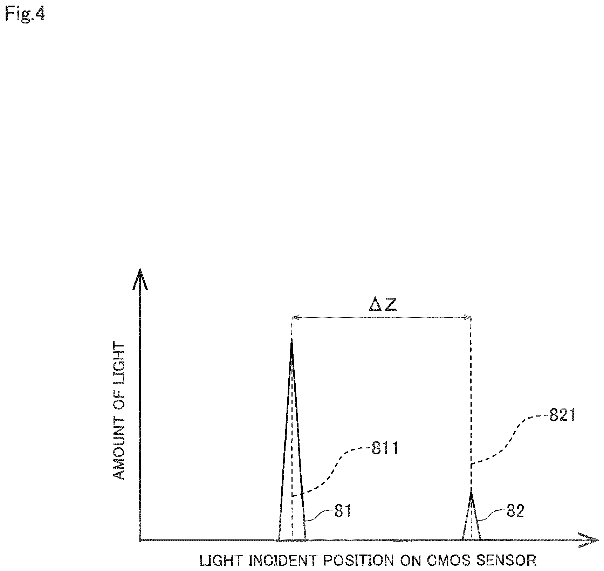

Fig. 4 is a graph showing a distribution of the amount of reflected light entering the CMOS sensor 31. The abscissa of Fig. 4 represents the incident position of the reflected light upon the CMOS sensor 31. The ordinate of Fig. 4 represents the amount of light. As shown in Fig. 4, the CMOS sensor 31 detects the light amounts of the first reflected light beam 81 and the second reflected light beam 82 at each position. Thus, a first peak position 811 at which the light amount of the first reflected light beam 81 is maximized and a second peak position 821 at which the light amount of the second reflected light beam 82 is maximized are detected.

The controller 71 is implemented by a computer 70 to be described later. The controller 71 controls the operations of the light irradiator 20 and the detector 30. The controller 71 also calculates the position of the second bottom surface 92 from the first peak position 811 of the first reflected light beam 81 and the second peak position 821 of the second reflected light beam 82 both detected by the detector 30.

### <3. Configuration of Image Acquisition Apparatus>

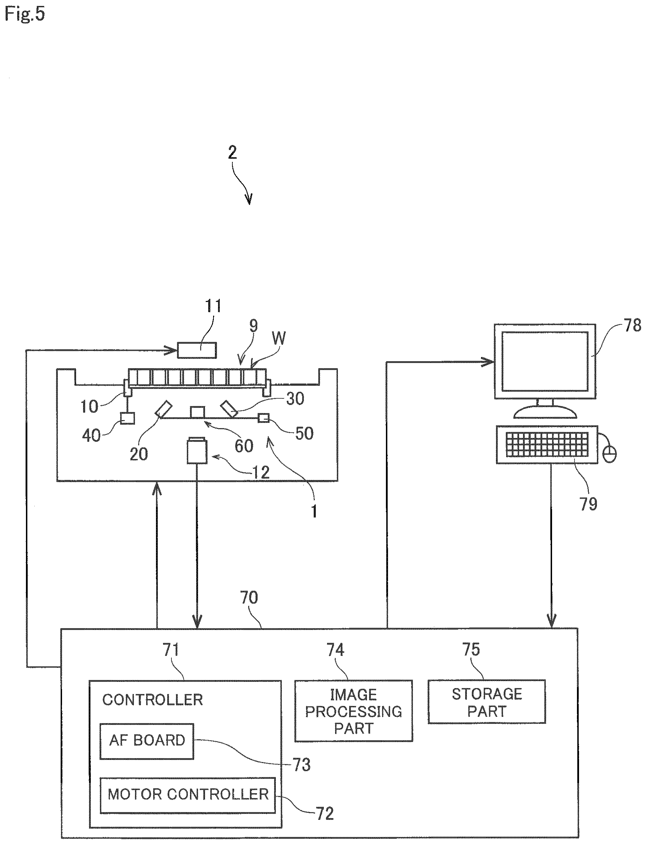

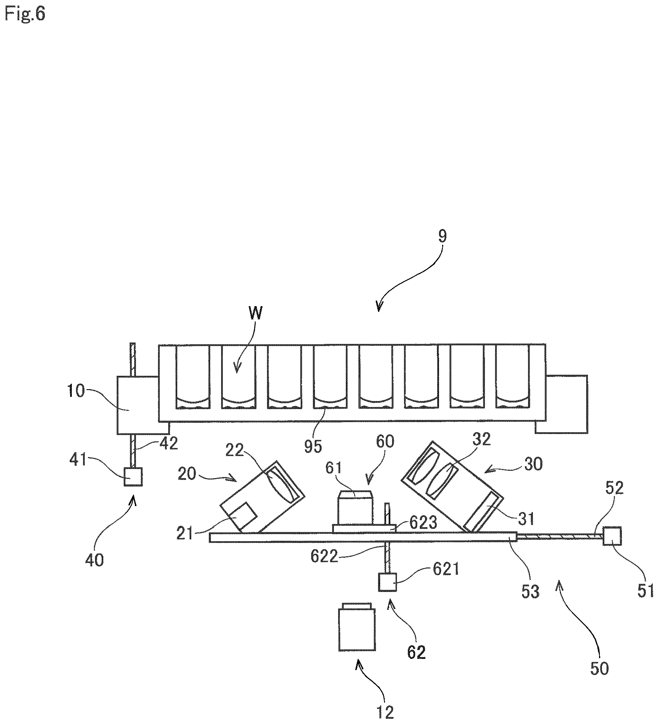

Next, an image acquisition apparatus 2 including the aforementioned bottom surface position detection apparatus 1 will be described. Fig. 5 is a diagram conceptually showing a configuration of the image acquisition apparatus 2 according to one embodiment of the present invention. Fig. 6 is a view showing the well plate 9 and its vicinities in the image acquisition apparatus 2.

This image acquisition apparatus 2 is an apparatus for processing image data obtained by photographing the well plate 9. The image acquisition apparatus 2 is used, for example, for a screening step for narrowing down compounds serving as candidates for medical and pharmaceutical products in the field of research and development of the medical and pharmaceutical products. An operator for the screening step uses images acquired by the image acquisition apparatus 2 to compare and analyze the image data about each well plate 9, thereby verifying the effects of the compounds added to the culture solution 94. It should be noted that the image acquisition apparatus 2 may be used for development of cells such as pluripotent stem cells themselves.

As shown in Figs. 5 and 6, the image acquisition apparatus 2 according to the present embodiment includes the aforementioned bottom surface position detection apparatus 1, a transmitted illumination part 11, an imaging part 12, a vertical movement mechanism 40, a horizontal movement mechanism 50, an autofocus mechanism 60, a display device 78, an input device 79, and the computer 70. The bottom surface position detection apparatus 1, the transmitted illumination part 11, the imaging part 12, the display device 78, and the input device 79 are electrically connected to the computer 70.

The transmitted illumination part 11 is disposed over the well plate 9 held by the holder 10. The imaging part 12 is disposed under the well plate 9. The imaging part 12 includes a camera having an image-forming lens and an imaging device such as CCD, CMOS and other imaging devices, for example. For taking images of the well plate 9, white light is directed from the transmitted illumination part 11 toward part of the well plate 9. Then, the white light is transmitted through the well plate 9 to travel to under the well plate 9. The imaging part 12 takes images of the part of the well plate 9 through an objective lens 61 to be described later.

The transmitted illumination part 11 may be any device which directs light toward the well plate 9. The transmitted illumination part 11 may have a light source disposed in a position deviated from over the well plate 9, and configured to direct light therefrom through an optical system such as a mirror onto the well plate 9. Also, the transmitted illumination part 11 may be disposed under the well plate 9, whereas the imaging part 12 be disposed over the well plate 9. Further, the transmitted illumination part 11 may be configured to cause light reflected from the well plate 9 to enter the imaging part 12. The light directed toward the well plate 9 is not limited to white light.

The vertical movement mechanism 40 is a mechanism for moving the holder 10 in a vertical direction. As shown in Fig. 6, the vertical movement mechanism 40 includes a motor 41 serving as a driving source and a ball screw 42 for transmitting the driving force of the motor 41. The ball screw 42 has one end connected to the motor 41. The holder 10 is mounted to the ball screw 42 so as to mesh with a spiral thread groove provided in an outer peripheral surface of the ball screw 42. By driving the motor 41, the ball screw 42 rotates about its axis. Thus, the holder 10 and the well plate 9 move in a vertical direction along the ball screw 42. That is, the rotary motion of the motor 41 is converted into the vertical linear motion of the holder 10 by way of the ball screw 42.

The horizontal movement mechanism 50 is a mechanism for moving the light irradiator 20, the detector 30, and the autofocus mechanism 60 in a horizontal direction. The horizontal movement mechanism 50 includes a motor 51 serving as a driving source, a ball screw 52 for transmitting the driving force of the motor 51, and a coupling member 53. The ball screw 52 has one end connected to the motor 51. The light irradiator 20, the detector 30, the imaging part 12, and the autofocus mechanism 60 are connected to each other by the coupling member 53. The coupling member 53 is mounted to the ball screw 52 so as to mesh with a spiral thread groove provided in an outer peripheral surface of the ball screw 52. By driving the motor 51, the ball screw 52 rotates about its axis. Thus, the light irradiator 20, the detector 30, the imaging part 12, and the autofocus mechanism 60 connected to each other by the coupling member 53 move in a horizontal direction along the ball screw 52 while maintaining the mutual positional relationship therebetween relative to each other. That is, the rotary motion of the motor 51 is converted into the horizontal linear motion of the holder 10 by way of the ball screw 52.

The autofocus mechanism 60 is a mechanism for focusing the imaging part 12 on the cells 95 in the wells W. The autofocus mechanism 60 according to the present embodiment has a movable part including the objective lens 61 and an AF shaft movement mechanism 62. The objective lens 61 is an optical system for focusing the imaging part 12 on the cells 95 in the wells W of the well plate 9. The autofocus mechanism 60 including the objective lens 61 may be part of the imaging part 12.

The AF shaft movement mechanism 62 is a mechanism for moving the objective lens 61 in a vertical direction relative to the coupling member 53. The AF shaft movement mechanism 62 includes a motor 621 serving as a driving source, a ball screw 622, and a support base 623. The objective lens 61 is fixed to the support base 623. The ball screw 622 extends in a vertical direction, and has one end connected to the motor 621. The support base 623 is mounted to the ball screw 622 so as to mesh with a spiral thread groove provided in an outer peripheral surface of the ball screw 622. By driving the motor 621, the ball screw 622 rotates about its axis. Thus, the support base 623 moves along the ball screw 622. That is, the rotary motion of the motor 621 is converted into the vertical linear motion of the objective lens 61 fixed to the support base 623 by way of the ball screw 622. The AF shaft movement mechanism 62 is capable of moving the support base 623 at a smaller pitch than the vertical movement mechanism 40. However, the AF shaft movement mechanism 62 is narrower in range of movement than the vertical movement mechanism 40.

The display device 78 is a section for displaying the images acquired and processed by the image acquisition apparatus 2. A liquid crystal display, for example, is used as the display device 78. The input device 79 is a section for inputting various commands to the computer 70. A keyboard and a mouse, for example, are used as the input device 79. A user of the image acquisition apparatus 2 may manipulate the input device 79 to input various commands to the computer 70 while viewing the display device 78.

Both the functions of the display device 78 and the functions of the input device 79 may be implemented by a single device such as a touch panel display device.

The computer 70 is an information processing device including a CPU, a memory, and the like. As shown in Fig. 5, the computer 70 includes the aforementioned controller 71, an image processing part 74, and a storage part 75. The computer 70 operates in accordance with previously set computer programs, input signals, and various data to implement the functions of the controller 71 and the image processing part 74. The controller 71 controls the operations of the transmitted illumination part 11, the imaging part 12, the laser diode 21, the CMOS sensor 31, and the motors 41, 51 and 621 described above, whereby an image acquisition process to be described later is executed.

The controller 71 according to the present embodiment includes a motor controller 72 and an AF board 73. For setting an AF reference position to be described later, the controller 71 controls the vertical movement mechanism 40 by way of the motor controller 72. This causes the holder 10 to move in a vertical direction. For an auto focus process to be described later, the controller 71 controls the AF shaft movement mechanism 62 by way of the AF board 73. This causes the objective lens 61 to move in a vertical direction. A detection signal from the detector 30 is read by the computer 70 by way of the AF board 73. In this manner, the single AF board 73 performs both the process of reading the detection signal from the detector 30 and the process of controlling the AF shaft movement mechanism 62 in the present embodiment. This allows the execution of the autofocus process on the wells W in a short time.

The image processing part 74 processes images of the cells 95 in the wells W, based on the acquired image data. The processed images are displayed on the display device 78. The image processing part 74 may have the function of performing image processing, based on commands inputted by the input device 79, and the function of automatically performing a screening process, based on acquired images.

The storage part 75 is a section for storing therein various data to be handled in the image acquisition apparatus 2. The storage part 75 is implemented by a storage device including a hard disk drive, a RAM, and the like, for example. The storage part 75 may be part of hardware constituting the computer 70 or be an external storage device connected to the computer 70.

### <4. Image Acquisition Process>

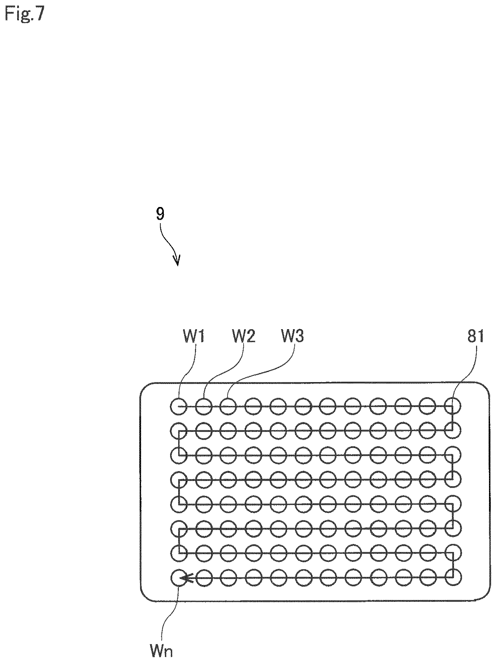

Next, the image acquisition process by means of the image acquisition apparatus 2 will be described. Fig. 7 is a view showing a course of the process of scanning the well plate 9 by means of the image acquisition apparatus 2. The image acquisition apparatus 2 moves the light irradiator 20, the detector 30, the imaging part 12, and the autofocus mechanism 60 in a horizontal direction. Then, the image acquisition apparatus 2 performs the scanning process for taking images of the cells 95 in the wells W by means of the imaging part 12 while performing the autofocus process on the wells W (W1 to Wn) of the well plate 9 in the order of W1, W2, W3, ..., and Wn as indicated by the arrow in Fig. 7. Other courses and orders than those shown in Fig. 7 may be used for the scanning process by means of the image acquisition apparatus 2.

The controller 71 detects the position of the second bottom surface 92 from the first reflected light beam 81 and the second reflected light beam 82 which enter the CMOS sensor 31. As shown in Fig. 4, the light amount of the second reflected light beam 82 entering the CMOS sensor 31 is smaller than that of the first reflected light beam 81. Based on this, the controller 71 determines the second reflected light beam 82 and the second peak position 821. A difference ΔZ between the first peak position 811 and the second peak position 821 is varied depending on the thickness 93 of the bottom plate portion 90 of the well plate 9. Specifically, the difference ΔZ increases as the thickness 93 of the bottom plate portion 90 increases, and the difference ΔZ decreases as the thickness 93 of the bottom plate portion 90 decreases. The controller 71 detects the position of the second bottom surface 92 from the second peak position 821 and the difference ΔZ.

The autofocus process is the process of automatically focusing the imaging part 12 on the cells 95 in each of the wells W, and is performed for each of the wells W. Based on the detected position of the second bottom surface 92, the controller 71 controls the AF shaft movement mechanism 62 of the autofocus mechanism 60 to perform the autofocus process.

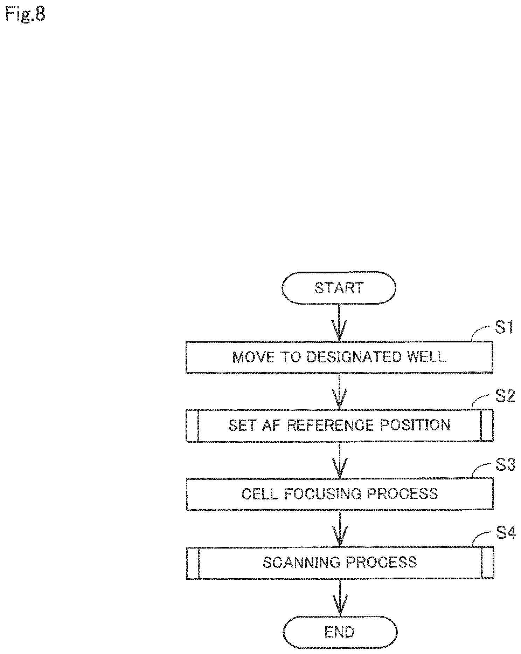

Fig. 8 is a flow diagram showing a general procedure for the image acquisition process by means of the image acquisition apparatus 2. For the execution of the scanning process, a user initially selects one of the wells W. The controller 71 moves the light irradiator 20, the detector 30, the imaging part 12, and the autofocus mechanism 60 to over and under the selected one well (referred to hereinafter as a designated well Ws) (Step S1). In the example of Fig. 7, the well W1 is the designated well Ws. Next, the controller 71 sets the AF reference position to be described later (Step S2). Subsequently, the controller 71 controls the AF shaft movement mechanism 62 to perform a cell focusing process for bringing the cells 95 in the wells W into focus (Step S3). Then, while controlling the horizontal movement mechanism 50 and performing the autofocus process on the cells 95 in the wells W to be imaged, the controller 71 performs the scanning process for sequentially taking images of the cells 95 in the wells W by means of the imaging part 12 (Step S4).

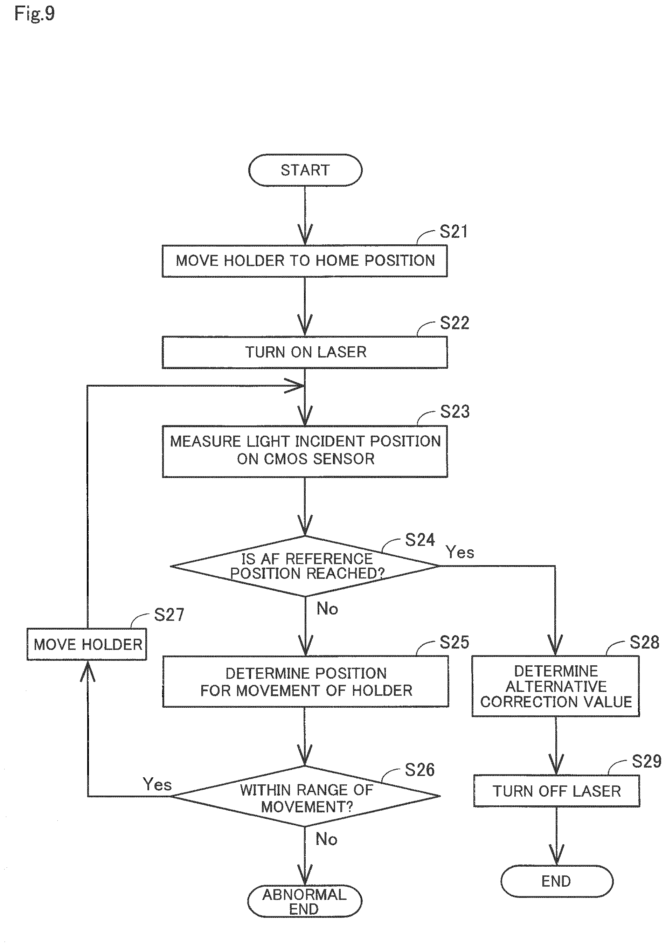

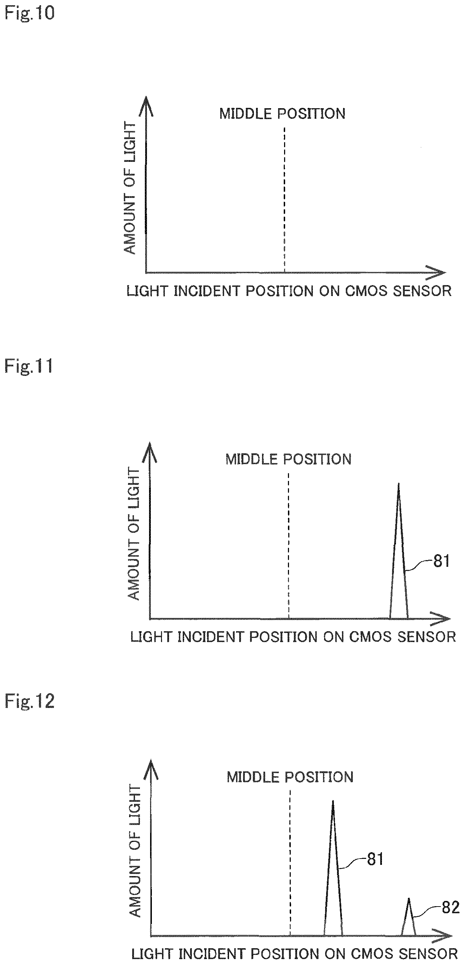

Fig. 9 is a flow diagram showing details on an AF reference position setting process. Figs. 10 to 13 are graphs showing exemplary distributions of the amount of light detected by the CMOS sensor 31 in the AF reference position setting process.

The AF (autofocus) reference position is the position of the second bottom surface 92 relative to the objective lens 61 at the time that the imaging part 12 is focused on the second bottom surface 92 of the designated well Ws selected by the user. The AF reference position setting process is the process of adjusting the vertical position of the holder 10 so as to focus the imaging part 12 on the second bottom surface 92 of the designated well Ws. The AF reference position serves as a reference position for the autofocus process in other wells W.

In the AF reference position setting process (Step S2), the controller 71 initially controls the vertical movement mechanism 40 to move the holder 10 to its home position (Step S21), as shown in Fig. 9. The home position shall be a position sufficiently upwardly spaced apart from the position of the holder 10 at the AF reference position. Next, the controller 71 starts the laser diode 21 to cause the laser diode 21 to direct a laser light beam toward the bottom portion of the well plate 9 (Step S22), thereby causing the CMOS sensor 31 to measure the incident position of the reflected light (Step S23).

Subsequently, the controller 71 judges whether the position of the holder 10 reaches the AF reference position or not (Step S24). The controller 71 judges that the positions of the holder 10 and the objective lens 61 relative to each other does not reach the AF reference position (No in Step S24) when the first reflected light beam 81 and the second reflected light beam 82 do not enter the CMOS sensor 31, as shown in Fig. 10. In this case, the controller 71 determines the next position for movement of the holder 10 (Step S25). The next position for movement shall be a position downwardly spaced a predetermined moving distance (e.g., a half of the detection range of the CMOS sensor 31) apart from the current position of the holder 10. Then, if the determined position for movement is within the range of movement of the vertical movement mechanism 40 (Step S26), the controller 71 moves the holder 10 downwardly (Step S27). On the other hand, if the determined position for movement is outside the range of movement of the vertical movement mechanism 40, the controller 71 judges that an anomaly occurs to terminate the AF reference position setting process.

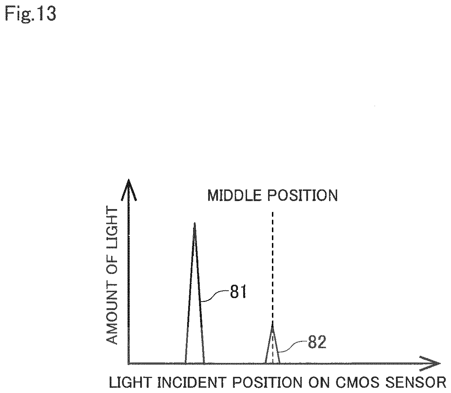

By repeating Steps S23 to S27, the CMOS sensor 31 detects the first reflected light beam 81 in the course of time, as shown in Fig. 11. By further repeating Steps S23 to S27, the CMOS sensor 31 further detects the second reflected light beam 82 as shown in Fig. 12. After the CMOS sensor 31 detects both the first reflected light beam 81 and the second reflected light beam 82, the controller 71 determines an interval between the second peak position 821 of the second reflected light beam 82 and the middle position of the CMOS sensor 31. Then, the controller 71 determines the position downwardly spaced a distance corresponding to this interval as the next position for movement of the holder 10 (Step S25) to move the holder 10 to this position for movement (Step S27). Specifically, the holder 10 is moved so that the second peak position 821 of the second reflected light beam 82 coincides with the middle position of the CMOS sensor 31. Then, the controller 71 judges that the position of the holder 10 reaches the AF reference position (Yes in Step S24) if the second peak position 821 of the second reflected light beam 82 reaches the middle position of the CMOS sensor 31, as shown in Fig. 13. Upon judging that the position of the holder 10 reaches the AF reference position, the controller 71 determines an alternative correction value to be described later (Step S28). Thereafter, the controller 71 stops the laser diode 21 (Step S29), and completes the AF reference position setting process (Step S2).

At the completion of Step S2, the imaging part 12 is focused on the second bottom surface 92 of the designated well Ws selected by the user. Subsequently, the controller 71 performs the cell focusing process in Step S3. In the cell focusing process, the focal position of the imaging part 12 is moved from the second bottom surface 92 to the cells 95 on the second bottom surface 92. Specifically, bracket photographing is performed a plurality of times on images of the designated well Ws while slightly vertically displacing the position of the objective lens 61, for example. Based on the plurality of obtained images, the position of the objective lens 61 optimum for photographing is determined. Then, the objective lens 61 is located at that position.

Next, the aforementioned alternative correction value will be described. The alternative correction value is a difference between the first peak position 811 and the second peak position 821 which are obtained in the AF reference position setting process. The autofocus process is performed on the wells W while the light irradiator 20, the detector 30, and the imaging part 12 are moved in a horizontal direction. Thus, there are cases in which the second reflected light beam 82 does not enter the CMOS sensor 31, such as a case in which the laser light beam enters a region lying between adjacent ones of the wells W. To prevent this, the alternative correction value is previously stored in Step S2. If the second reflected light beam 82 does not enter the CMOS sensor 31, the second peak position 821 is presumed from the first peak position 811 of the first reflected light beam 81 entering the CMOS sensor 31, based on the previously stored alternative correction value. This allows the imaging part 12 to be focused on the cells 95 by controlling the AF shaft movement mechanism 62, based on the presumed second peak position 821, even if the second reflected light beam 82 does not enter the CMOS sensor 31.

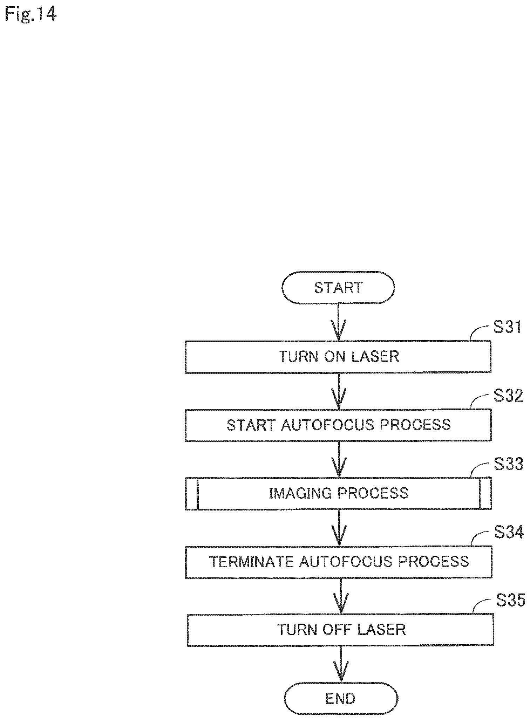

Next, the scanning process in Step S4 will be described. Fig. 14 is a flow diagram showing a procedure for the scanning process. For execution of the scanning process, the controller 71 initially starts the laser diode 21 (Step S31). Next, the controller 71 instructs the AF board 73 to start the autofocus process (Step S32). The controller 71 controls the horizontal movement mechanism 50 to move the light irradiator 20, the detector 30, and the imaging part 12 in a horizontal direction on the course shown in Fig. 7, for example. Then, while performing the autofocus process for each of the wells W, the controller 71 causes the imaging part 12 to perform an imaging process for imaging the cells 95 in each well W (Step S33). Upon completion of the imaging process of all of the wells W to be observed, the controller 71 terminates the autofocus process (Step S34). Thereafter, the controller 71 stops the laser diode 21 (Step S35). This completes the scanning process by means of the image acquisition apparatus 2.

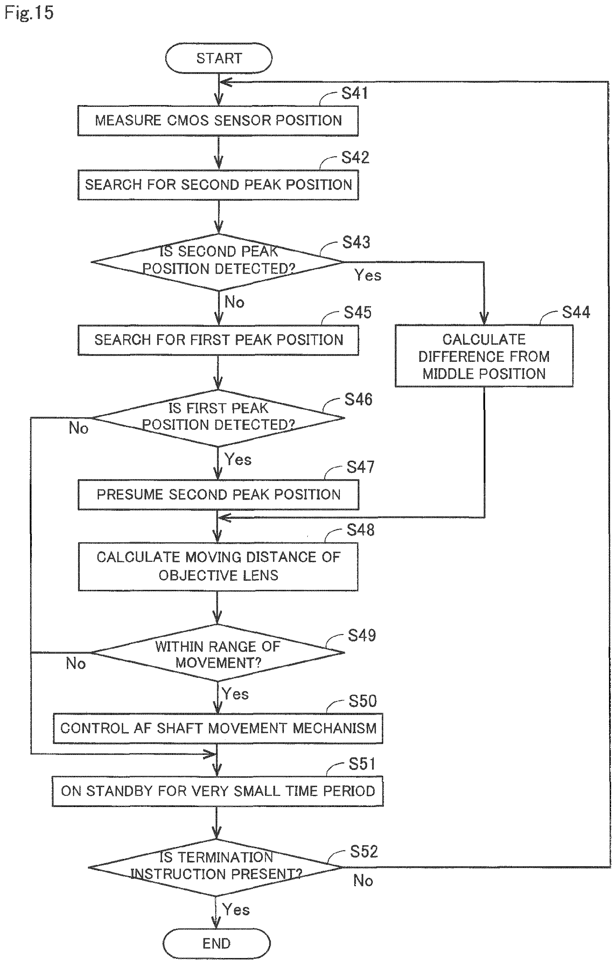

Fig. 15 is a flow diagram showing details on the autofocus process performed in the imaging process in Step S33. For the execution of the autofocus process, the controller 71 initially controls the CMOS sensor 31 to read the positions of the reflected light beams entering the CMOS sensor 31 (Step S41). Next, the controller 71 searches for the second peak position 821 of the second reflected light beam 82 from the distribution of the light amount of the laser light beam entering the CMOS sensor 31 (Step S42). Then, if the second peak position 821 is detected (Step S43), the controller 71 calculates the difference of the second peak position 821 from the middle position of the CMOS sensor 31 (Step S44). Based on the difference, the controller 71 calculates the amount of displacement of the second bottom surface 92 from the AF reference position.

On the other hand, if the second peak position 821 is not detected in Step S43, the controller 71 searches for the first peak position 811 of the first reflected light beam 81 entering the CMOS sensor 31 (Step S45). Then, if the first peak position 811 is detected (Step S46), the controller 71 adds the aforementioned alternative correction value to the first peak position 811 to presume the second peak position 821 (Step S47). Then, the controller 71 calculates the amount of displacement of the second bottom surface 92 from the AF reference position, based on the difference of the presumed second peak position 821 from the middle position of the CMOS sensor 31.

The difference calculated in Step S44 or Step S47 corresponds to a displacement of the position of the second bottom surface 92 between each well W to be observed and the designated well Ws selected by the user. The controller 71 calculates the moving distance of the objective lens 61 by means of the AF shaft movement mechanism 62 for keeping up with the displacement (Step S48). Then, the controller 71 judges whether the moving distance of the objective lens 61 calculated in Step S48 is within the range of movement of the AF shaft movement mechanism 62 or not (Step S49). If the calculated moving distance is within the range of movement, the controller 71 controls the AF shaft movement mechanism 62 to move the objective lens 61 through the moving distance (Step S50). Thus, the imaging part 12 is focused on the cells 95 in each well W to be observed.

Thereafter, the controller 71 places the image acquisition apparatus 2 on standby for a very small time period (e.g., tens of milliseconds) (Step S51), and judges whether a termination instruction has been inputted or not (Step S52). If the termination instruction is absent, the procedure returns to Step S41, and the aforementioned processes in Steps S41 to S52 are repeated. On the other hand, if the termination instruction is present, the autofocus process is terminated.

In this manner, the image acquisition apparatus 2 according to the present embodiment performs the scanning process while detecting the position of the second bottom surface 92 and the displacement of the position of the second bottom surface 92 between the wells W. Thus, the image acquisition apparatus 2 is capable of focusing the imaging part 12 on the cells 95 in the wells W of the well plate 9 even if the bottom surface of the well plate 9 is curved or the thickness 93 of the bottom plate portion 90 of the well plate 9 is uneven. This achieves the acquisition of sharp images in the wells W.

In particular, the light irradiator 20 according to the present embodiment directs the laser light beam obliquely upwardly toward the bottom portion of the well plate 9. Also, the detector 30 detects the laser light beam reflected obliquely downwardly from the bottom portion of the well plate 9. Such an arrangement increases the difference ΔZ between the first peak position 811 and the second peak position 821. Thus, the position of the second bottom surface 92 is calculated more precisely.

### <5. Modifications>

While the one embodiment according to the present invention has been described hereinabove, the present invention is not limited to the aforementioned embodiment.

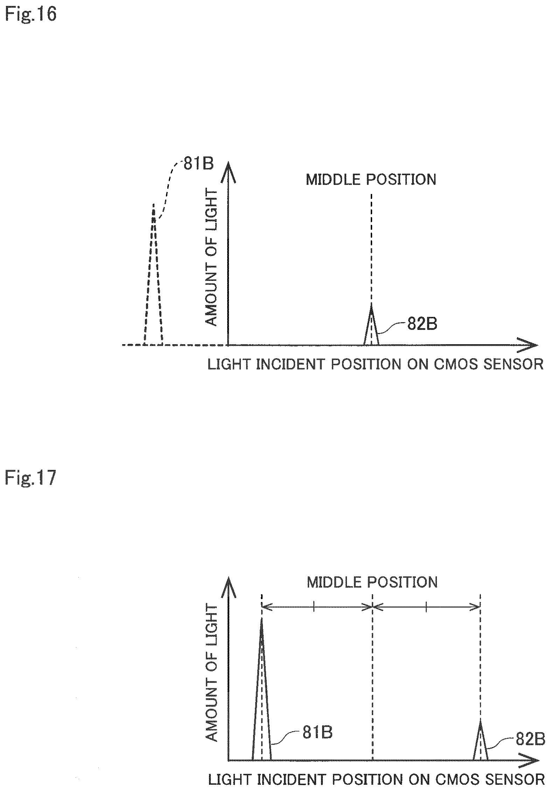

Fig. 16 is a graph showing a relationship between a distribution of the light amounts of a first reflected light beam 81B and a second reflected light beam 82B and the position of the CMOS sensor in the case where the thickness of the bottom plate portion 90 of the well plate 9 is large. In the case where the thickness of the bottom plate portion 90 of the well plate 9 is large, the first reflected light beam 81B does not enter the CMOS sensor when the second peak position of the second reflected light beam 82B is adjusted to the middle position of the CMOS sensor. In such a case, a middle position between the first and second peak positions may be adjusted to the middle position of the CMOS sensor, as shown in Fig. 17. In the auto focus process, the second peak position shown in Fig. 17 may be defined as a standard position, and the difference of the second peak position from the standard position may be calculated. Thus, the autofocus process is performed on the plurality of wells while the displacement of the second bottom surface is detected even in the case where the thickness of the bottom plate portion 90 of the well plate 9 is large.

The light irradiator according to the aforementioned embodiment directs the laser light beam obliquely upwardly toward the bottom portion of the well plate. Also, the detector detects the laser light beam reflected obliquely downwardly from the bottom portion of the well plate. However, the light irradiator may direct the laser light beam in the same lens barrel as the objective lens substantially upwardly toward the bottom portion of the well plate. Also, the detector may detect the laser light beam reflected substantially downwardly from the bottom portion of the well plate in the same lens barrel as the objective lens.

The vessel according the aforementioned embodiment is the plastic molded well plate. However, the vessel may be a petri dish made of glass, resin, and the like and having a single well. Alternatively, the vessel may be vessels or containers of other various types.

The vertical movement mechanism, the horizontal movement mechanism, and the AF shaft movement mechanism according to the aforementioned embodiment include ball screw mechanisms. However, the vertical movement mechanism, the horizontal movement mechanism, and the AF shaft movement mechanism may include other mechanisms (e.g., linear motor mechanisms) than the ball screw mechanisms.

In the aforementioned embodiment, the imaging part is focused on the second bottom surface by the vertical movement mechanism, and is thereafter focused on the cells in the wells by the autofocus mechanism. However, if the autofocus mechanism is able to also serve as the vertical movement mechanism by increasing the range of movement of the autofocus mechanism, the vertical movement mechanism may be dispensed with. In other words, the vertical movement mechanism and the autofocus mechanism according to the present invention may be implemented by the same mechanism.

In the aforementioned embodiment, the horizontal position of the well plate is fixed, whereas the light irradiator, the detector, the imaging part, and the autofocus mechanism are moved in a horizontal direction. Alternatively, the well plate may be moved in a horizontal direction while the horizontal positions of the light irradiator, the detector, the imaging part, and the autofocus mechanism are fixed. It is, however, desirable that the horizontal position of the well plate is fixed as in the aforementioned embodiment for the purpose of suppressing the culture solution sloshing around in the wells.

The light source in the light irradiator includes the laser diode according to the aforementioned embodiment. However, the light source in the light irradiator may include other light emitting devices such as LEDs.

The detector includes the CMOS sensor according to the aforementioned embodiment. However, the detector may include other light-receiving devices such as CCD sensors.

The configurations of the details on the bottom surface position acquisition apparatus and the image acquisition apparatus may differ from those shown in the figures of the present invention. The components described in the aforementioned embodiment and in the various modifications may be consistently combined together, as appropriate.

### Reference Signs List

- 1: Bottom surface position detection apparatus
- 2: Image acquisition apparatus
- 9: Well plate
- 10: Holder
- 11: Transmitted illumination part
- 12: Imaging part
- 20: Light irradiator
- 21: Laser diode
- 22: Lens
- 30: Detector
- 31: CMOS sensor
- 32: Lenses
- 40: Vertical movement mechanism
- 41: Motor
- 42: Ball screw
- 50: Horizontal movement mechanism
- 51: Motor
- 52: Ball screw
- 53: Coupling member
- 60: Autofocus mechanism
- 61: Objective lens
- 62: AF shaft movement mechanism
- 70: Computer
- 71: Controller
- 72: Motor controller
- 73: AF board
- 74: Image processing part
- 75: Storage part
- 78: Display device
- 79: Input device
- 81: First reflected light beam
- 82: Second reflected light beam
- 90: Bottom plate portion
- 91: First bottom surface
- 92: Second bottom surface
- 93: Thickness of bottom plate portion
- 94: Culture solution
- 95: Cells
- 621: Motor
- 622: Ball screw
- 623: Support base
- 811: first peak position
- 821: Second peak position
- W: Wells
- Ws: Designated well
- ΔZ: Difference

## Claims

1. A bottom surface position detection apparatus for detecting a bottom surface position of a light-permeable vessel, comprising:
a holder for horizontally holding said vessel;
a light irradiator for irradiating a bottom portion of said vessel with a light beam;
a detector for detecting the light beam emitted from said light irradiator and then reflected from said vessel; and
a controller,
said vessel having at least one vertically depressed well,
said detector detecting a first reflected light beam reflected from a first bottom surface that is a bottom surface of said vessel and a second reflected light beam reflected from a second bottom surface that is a bottom surface of said well,
said controller calculating the position of said second bottom surface, based on a first peak position of the first reflected light beam and a second peak position of the second reflected light beam both detected by said detector.

2. The bottom surface position detection apparatus according to Claim 1, wherein
said light irradiator directs the light beam obliquely upwardly toward the bottom portion of said vessel.

3. An image acquisition apparatus for acquiring an image of biological cells disposed in said well of said vessel, comprising:
a bottom surface position detection apparatus as recited in Claim 1 or 2;
an imaging part for taking an image of the cells in said well;
an image processing part for processing the image taken by said imaging part;
a vertical movement mechanism for moving said holder in a vertical direction;
an autofocus mechanism for adjusting a focal position of said imaging part with respect to said cells; and
a horizontal movement mechanism for moving said imaging part, said light irradiator, and said detector in a horizontal direction relative to said vessel,
said controller controlling said vertical movement mechanism while referring to a detection result of said detector, and controlling said autofocus mechanism after the focal position of said imaging part is adjusted to said second bottom surface.

4. The image acquisition apparatus according to Claim 3, wherein
said controller stores the position of said second bottom surface at a certain position as an autofocus reference position to control said autofocus mechanism, based on said autofocus reference position and said second peak position detected from said detector, after controlling said horizontal movement mechanism.

5. The image acquisition apparatus according to Claim 3 or 4, wherein
said controller stores a difference between said first peak position and said second peak position as an alternative correction value to control said autofocus mechanism, based on said first peak position detected from said detector and said alternative correction value, after controlling said horizontal movement mechanism.

6. A bottom surface position detection method for detecting a bottom surface position of a light-permeable vessel including at least one vertically depressed well, comprising the steps of:
a) horizontally holding said vessel;
b) irradiating a bottom portion of said vessel with a light beam from a light irradiator;
c) detecting the light beam impinging upon the bottom portion of said vessel and then reflected from said vessel by means of a detector; and
d) calculating the bottom surface position of said vessel,
said step c) detecting a first reflected light beam reflected from a first bottom surface that is a bottom surface of said vessel and a second reflected light beam reflected from a second bottom surface that is a bottom surface of said well,
said step d) calculating the position of said second bottom surface, based on a first peak position of the first reflected light beam and a second peak position of the second reflected light beam both detected by said step c).

7. The bottom surface position detection method according to Claim 6, wherein
said step c) directs the light beam obliquely upwardly toward the bottom portion of said vessel.

8. An image acquisition method including a bottom surface position detection method as recited in Claim 6 or 7 and for acquiring an image of biological cells disposed in said well of said vessel, comprising the steps of:
e) taking an image of the cells in said well by means of an imaging part; and
f) processing the image taken by said imaging part,
said step d) moving said vessel in a vertical direction while referring to a detection result in said step c), and adjusting a focal position of said imaging part with respect to said cells by means of an autofocus mechanism after the focal position of said imaging part is adjusted to said second bottom surface.

9. The image acquisition method according to Claim 8, wherein
said step d) stores the position of said second bottom surface at a certain position as an autofocus reference position to control said autofocus mechanism, based on said autofocus reference position and said second peak position detected from said detector, after controlling said horizontal movement mechanism.

10. The image acquisition method according to Claim 8 or 9, wherein
said step d) includes the step of storing a difference between said first peak position and said second peak position as an alternative correction value to control said autofocus mechanism, based on said first peak position detected in said step c) and said alternative correction value.
